(19) Europäisches Patentamt
European Patent Office
Office européen des brevets

(11) Veröffentlichungsnummer: **0 288 813**
**B1**

(12) # EUROPÄISCHE PATENTSCHRIFT

(45) Veröffentlichungstag der Patentschrift: **14.11.90**

(51) Int. Cl.[5]: **C09K 19/58,** C07D 317/24, C07D 405/12, C07D 409/12, G02F 1/133

(21) Anmeldenummer: **88105777.2**

(22) Anmeldetag: **12.04.88**

(54) **Verwendung von optisch aktiven 1,3-Dioxolan-4-carbonsäureestern als Dotierstoffe in Flüssigkristallmischungen, diese enthaltende Flüssigkristallmischungen und neue optisch aktive 1,3-Dioxolan-4-carbonsäureester.**

(30) Priorität: **18.04.87 DE 3713273**

(43) Veröffentlichungstag der Anmeldung: **02.11.88 Patentblatt 88/44**

(45) Bekanntmachung des Hinweises auf die Patenterteilung: **14.11.90 Patentblatt 90/46**

(84) Benannte Vertragsstaaten: **AT BE CH DE ES FR GB IT LI NL SE**

(56) Entgegenhaltungen:
**DE-A- 3 604 898**
**US-A- 4 336 183**

**CHEMICAL ABSTRACTS, Band 88, Nr. 3, 16. JÄNNER 1978; Columbus, Ohio, USA LOK, C.M.; WARD; J.P.; VAN DORP, D.A. "The synthesis of chiral glycerides starting from D- and L-serine." Seite 566, Spalte 2, Zusammenfassung-Nr. 22 089e**
**CHEMICAL ABSTRACTS, Band 88, Nr. 3, 16. Jänner 1978, Columbus, Ohio, USA LOK, C.M.; WARD, J.P.; VAN DORP, D.A. "The synthesis of chiral glycerides starting fom D- and L-serine." Seite 566, Spalte 2, Zusammenfassung-Nr.22 094c**

(73) Patentinhaber: **HOECHST AKTIENGESELLSCHAFT, Postfach 80 03 20, D-6230 Frankfurt am Main 80(DE)**

(72) Erfinder: **Wingen, Rainer, Dr., Rotkäppchenweg 10, D-6234 Hattersheim am Main(DE)**
Erfinder: **Dübal, Hans-Rolf, Dr., Heuhohlweg 6, D-6240 Königstein/Taunus(DE)**
Erfinder: **Escher, Claus, Dr., Amselweg 3, D-6109 Mühltal(DE)**
Erfinder: **Hemmerling, Wolfgang, Dr., Billtalstrasse 32, D-6231 Sulzbach (Taunus)(DE)**
Erfinder: **Müller, Ingrid, Dr., Am Pfingstbrunnen 1, D-6238 Hofheim am Taunus(DE)**
Erfinder: **Ohlendorf, Dieter, Dr., Am Kühlen Grund 4, D-6237 Liederbach(DE)**

**EP 0 288 813 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, eher zu lang. Dieser Nachteil macht sich insbesondere dann bemerkbar, wenn eine große Anzahl von Bildpunkten angesteuert werden muß, wodurch die Herstellungskosten von Geräten, die größere Flächen enthalten, z.B.Videogeräte, Oszillographen oder Fernseh-, Radar-, EDV- oder Schreibautomaten-Bildschirme, zu hoch werden.

Neben den nematischen und cholesterischen Flüssigkristallen haben seit einigen wenigen Jahren in zunehmendem Maß auch optisch aktive, smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu optoelektrischen Schalt- oder Anzeigeelementen führt, die im Vergleich mit den herkömmlichen TN ("twisted nematic")-Zellen um bis zu einem Faktor 1000 schnellere Schaltzeigen haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrasts, sind ferroelektrische Flüssigkristalle grundsätzlich für die obengenannten Anwendungsgebiete, z.B. über eine Matrixansteuerung, gut geeignet.

Einen anderen elektro-optischen Effekt, der als elektrokliner Effekt bezeichnet wird, zeigen orthogonale chirale smektische Phasen, z.B. $S_A^*$, $S_A^*$, $S_A^*$. Dieser Effekt (S. Garoff und R.B. Meyer, Phys. Rev. Lett. **38**, 848 (1977)) besteht in einer feldinduzierten Neigung der Moleküle, deren Neigungswinkel $\theta$ sich proportional zum angelegten Feld ändert. Die Moleküle der orthogonalen Phasen können daher der Feldänderung kontinuierlich folgen, und sie können insbesondere einem Wechselfeld bis zu einer Grenzfrequenz $f_G$ folgen, während ferroelektrische Systeme jeweils beim Erreichen einer bestimmten Feldstärke ihren Neigungswinkel sprunghaft ändern und beibehalten, bis ein entsprechendes gegensinniges Feld angelegt wird (bistabiles Schalten).

Beide Effekte, der ferroelektrische, wie der elektrokline, lassen sich je nach ihren speziellen Eigenschaften zum Bau von elektro-optischen Schalt- und Anzeigeelementen ausnutzen. Man benötigt dazu entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen chirale, geneigte bzw. orthogonale smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A^*$- und $S_C^*$-Phase läßt sich erreichen, wenn die Phasenfolge der Flüssigkristallmischung mit abnehmender Temperatur lautet:

Isotrop $\rightarrow$ N* $\rightarrow$ $S_A^*$ $\rightarrow$ $S_C^*$.

Voraussetzung ist, daß der pitch (Ganghöhe der Helix) in der N*-Phase sehr groß (größer 10 µm) oder noch besser völlig kompensiert ist. (T. Matsumoto et al., S. 468-470, Proc. of the 6th Int. Display Research Conf., Japan Display, Sept. 30 - Okt. 2, 1986, Tokio, Japan; M. Murakami et al., ibid. S.344 - S.347). Dies erreicht man, indem man zu der chiralen Flüssigkristallmischung, die in der N*-Phase z.B. eine linksdrehende Helix aufweist, einen weiteren optisch aktiven Dotierstoff, der eine rechtsdrehende Helix induziert, in solchen Mengen hinzugibt, daß die Helix gerade kompensiert wird.

Es wurde nun gefunden, daß optisch aktive und mesogene 1,3-Dioxolan-4-carbonsäureester als Dotierstoffe in geneigt smektischen Flüssigkristallphasen schon bei geringen Zumischmengen zu kurzen Schaltzeiten und in orthogonal smektischen Flüssigkristallphasen zu hohen elektroklinen Koeffizienten führen. Besonders überraschend ist dabei, daß die Ganghöhe (pitch) der durch die Dotierung induzierten Helix so groß ist, daß eine Kompensation durch weitere Dotierstoffe nicht nötig ist.

Gegenstand der Erfindung ist daher die Verwendung von optisch aktiven und mesogenen 1,3-Dioxolan-4-carbonsäureestern als Dotierstoffe in Flüssigkristallsystemen. Gegenstand der Erfindung sind weiterhin Flüssigkristallsysteme, die diese 1,3-Dioxolan-4-carbonsäureester enthalten sowie neue optisch aktive 1,3-Dioxolan-4-carbonsäureester. Die gemäß der Erfindung als Dotierstoffe in Flüssigkristallmischungen zu verwendenden 1,3-Dioxolan-4-carbonsäureester entsprechen der allgemeinen Formel (I)

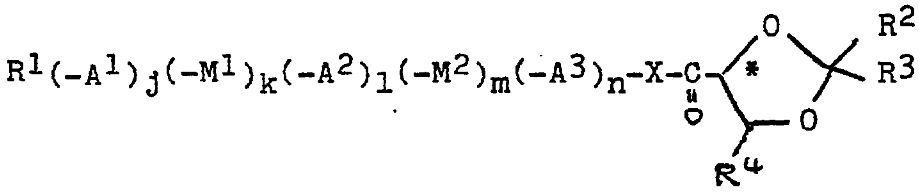 (I)

in der die Symbole folgende Bedeutung haben:

$R^1$

$R^1$

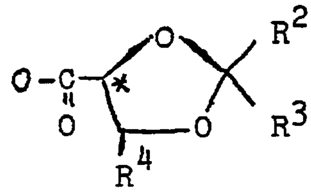 oder

oder

ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, wobei eine oder mehrere nicht benachbarte $-CH_2$-Gruppen durch

$-CH_2$-Gruppen durch -O-, -S-, $-\underset{\overset{\|}{O}}{C}-$, $-O-\underset{\overset{\|}{O}}{C}-$ und/oder

$-\underset{\overset{\|}{O}}{C}-O-$ und wobei ein oder mehrere H durch F, Cl, Br

oder CN ersetzt sein können

$R^2$ und $R^3$ jeweils H oder ein Alkylrest mit 1 bis 10 C-Atomen, wobei ein oder mehrere H durch F ersetzt sein können, oder $R^2$ und $R^3$ bilden zusammen mit dem C(2)-Atom des Dioxolanrings einen Cyclopentan-, Cyclohexan- oder Cycloheptanring

$R^4$ H oder ein Alkylrest mit 1 bis 10 oder ein Alkenylrest mit 2 bis 10 C-Atomen

j und l null, 1 oder 2

k und m null oder 1

n null, 1 oder 2

mit folgender Maßgabe: wenn j und/oder l = null sind, ist k = null; wenn n = null ist, ist m = null; die Summe j + l + n ist minimal 1 und maximal 3,

$-A^1$ und $-A^2$

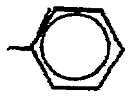 , 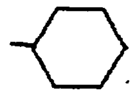 , 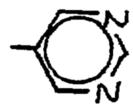 , 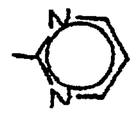 ,

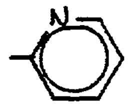 , 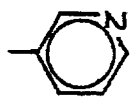 , 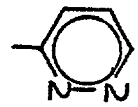 , 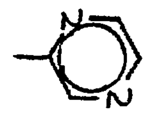 ,

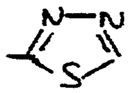 , 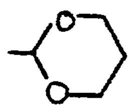 , 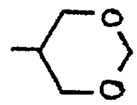 , 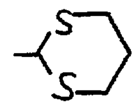 ,

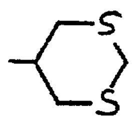 , 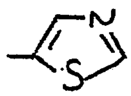 , 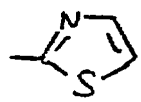 , 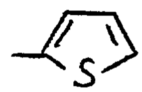 ,

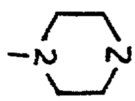

$-A^3$

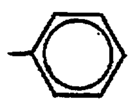 , 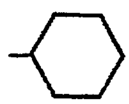 , 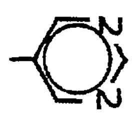 , 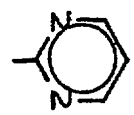 ,

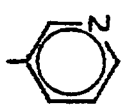 , 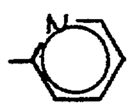 , 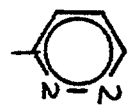

$-M^1$ und $-M^2$

$-\underset{\overset{\|}{O}}{C}-O$, $-O-\underset{\overset{\|}{O}}{C}$, $-CH_2CH_2$, $-CH{=}CH$, $-CH_2O$, $-OCH_2$

X O oder S.

In einer bevorzugten Ausführungsform haben die Symbole in der allgemeinen Formel (I) die folgende Bedeutung:

$R^1$ ein geradkettiger oder verzweigter Alkyl- oder Alkenylrest mit 4 bis l4 C-Atomen, der ein asymmetrisches C-Atom enthalten kann, oder wobei eine $-CH_2$-Gruppe durch

$-O-$, $-\underset{\overset{\|}{O}}{C}-$ oder $-\underset{\overset{\|}{O}}{C}-O-$

ersetzt sein känn, oder wobei ein der mehrere H durch F ersetzt sein können,

$R^2$,$R^3$,$R^4$ H oder ein Alkylrest mit 1 bis 5 C-Atomen oder $R^2$,$R^3$ zusammen mit dem C(2)-Atom des Dioxolanrings einen Cyclopentan- oder Cyclohexanring,

j und l null oder 1,

k,m,n null oder 1

$-M^1$, $-M^2$ 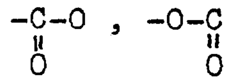

X O oder S.

In einer weiteren bevorzugten Ausführungsform werden 1,3-Dioxolan-4-carbonsäureester der allgemeinen Formel (IV) eingesetzt

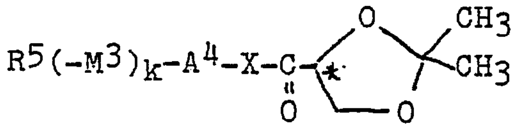 (IV)

worin bedeuten:

$R^5$ ein geradkettiger oder verzweigter Alkyl- oder Alkenylrest mit 6 bis 12 C-Atomen, der ein asymmetrisches C-Atom enthalten kann,

$-M^3$ 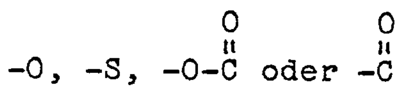

$-A^4$ 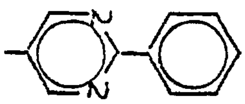 , 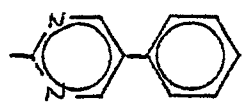 , 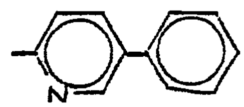 ,

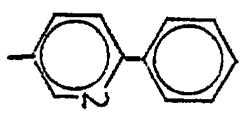 , 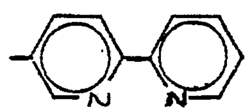 , 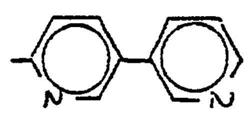 ,

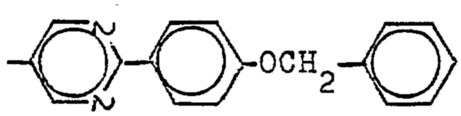 , 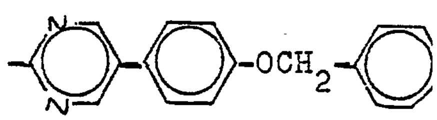

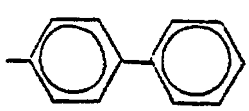 , 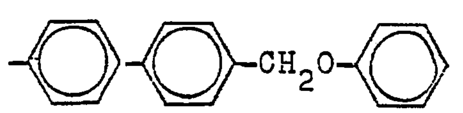 ,

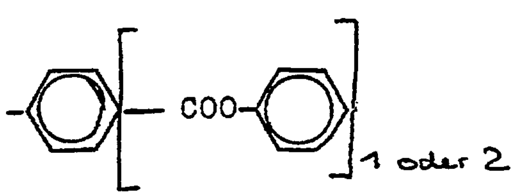

Zu den neuen Verbindungen der allgemeinen Formel (I), insbesondere (IV) gehören bevorzugt die in den Beispielen namentlich genannten Verbindungen.

Zur Herstellung der Verbindungen der allgemeinen Formel (I) werden mesogene Phenole bzw. Thiophenole der allgemeinen Formel (II)

$R^1(-A^1)_j(-M^1)_k(-A^2)_l(-M^2)_m(-A^3)_n$-X-H(II)

mit geeigneten Derivaten der 1,3-Dioxolan-4-carbonsäure (III), bevorzugt den Säurechloriden (mit Y = Cl),

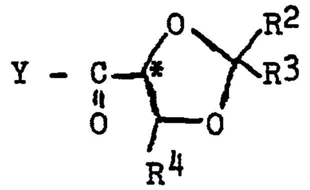

(III)

in Gegenwart äquivalenter oder überschüssiger Mengen organischer oder anorganischer Basen, gegebenenfalls unter Zuhilfenahme von Acylierungskatalysatoren in einem geeigneten Lösemittel umgesetzt und das Reaktionsprodukt durch geeignete Maßnahmen, z.B. Kristallisation, Filtration, chromatographische Trennverfahren, isoliert und gereinigt. Y ist dabei eine geeignete Abgangsgruppe wie Cl, Br, ONa oder vergleichbare Gruppen. Die zu verwendenden Phenole sind literaturbekannt. Methoden zur Herstellung in obigem Sinn geeigneter Derivate der 1,3-Dioxolän-4-carbonsäuren sind ebenfalls literaturbekannt, z. B. M. Angrick et al., Monatsheft für Chemie 116, 377 (1985); R. Dumont et al., Helv. Chim. Acta 66, 814 (1983), T. Sugiyama et al., Agric. Biol. Chem. 48, 1841 (1984) oder J. Jurczak et al., Tetrahedron 42, 447 (1986). Die Verknüpfung der beiden Bausteine (II) und (III) zu (I) kann nach an sich literaturbekannten Methoden erfolgen, wie beispielsweise von J.-M. Beau et al., Tetrahedron Letters 26, 6193 (1985), beschrieben.

Die Flüssigkristallmischungen gemäß der Erfindung bilden Flüssigkristall-Phasen und enthalten mindestens eine optisch aktive Verbindung der allgemeinen Formel (I).

Unter dem Begriff "Flüssigkristallphase" sind nematische, cholesterische, orthogonal smektische oder geneigt ("tilted")-smektische, insbesondere $S_A^*$-, $S_B^*$- und $S_C^*$-Phasen zu verstehen. Die Flüssigkristallmischungen bestehen aus 2 bis 20, vorzugsweise 2 bis 15 Komponenten, darunter mindestens eine der erfindungsgemäß beanspruchten chiralen Verbindungen.

Die anderen Bestandteile werden vorzugsweise ausgewählt aus den bekannten Verbindungen mit nematischen, cholesterischen und/oder smektischen, z.B. $S_A$-Phasen, und/oder geneigt smektischen Phäsen; dazu gehören beispielsweise Schiffsche Basen, Biphenyle, Terphenyle, Phenylcyclohexane, Cyclohexylbiphenyle, N, S oder O-haltige Heterocyclen, z.B. Pyrimidine, Zimtsäureester, Cholesterinester, verschiedene überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt. Insbesondere enthält die Flüssigkristall-Mischung neben mindestens einer der erfindungsgemäß beanspruchten optisch aktiven Verbindungen eine Esterverbindung mit $S_{C\text{-Phase}}$, z.B. einen Alkoxybenzoesäurephenylester, oder eine biaromatische Verbindung mit einem stickstoffhaltigen Heterocyclus, z.B. ein Alkylpyrimidinylalkoxy-benzol.

Von der oder den erfindungsgemäßen Verbindung(en) enthalten die Flüssigkristallmischungen im allgemeinen 0,05 bis 70 Gew.-%, insbesondere 0,1 bis 50 Gew.-%. Die erfindungsgemäßen Verbindungen sind insbesondere als Dotierstoffe für geneigt-smektische Flüssigkristallphasen geeignet, da sie diese in ferroelektrische Flüssigkristall-Phasen umwandeln; die Werte für die spontane Polarisation (Ps) bei 25°C liegen bei 10 Mol-% Dotierung im Bereich von etwa 8-14 nC/cm² und im Bereich von etwa 80-140 nC/cm² linear extrapoliert auf die reine Verbindung. Die Schaltzeiten der neuen Systeme liegen meistens deutlich unter 50 µs bei 10 Mol-% Dotierung, 25°C und einer Schaltspannung von ± 10 V/µm. Die erfindungsgemäßen Verbindungen können auch zur Erzielung des elektroklinen Effektes in orthogonalen smektischen Phasen ($S_A^*$, $S_B^*$, $S_E^*$) eingesetzt werden.

Beispiel 1

(R)-4-(2-n-Octyl-pyrimidin-5-yl)-phenyl-2,2-dimethyl- 1,3-dioxolan-4-carbonsäureester

[(I) mit $R^1 = C_8H_{17}$, j = 1,

j = 1, $-A^1$ = 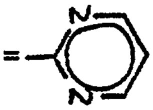,

k = 1 = m = null, n = 1,

$-A^3$ = 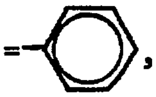,

X = O, $R^2 = R^3 = CH_3$, $R^4$ = H]

Eine Lösung von 3,2 g (11,3 mmol), 4-(2-n-Octyl-pyrimidin-5-yl)-phenol, 1,24 g (12,3 mmol) Triethylamin und 10 mg 4-Dimethylaminopyridin in 30 ml Tetrahydrofuran wird bei O°C innerhalb von 10 min. mit 1,86 g (11,3 mmol) 2,2-Dimethyl-1,3-dioxolan-4-carbonsäurechlorid versetzt. Nach dreistündigem Rühren bei O°C wird das Triethylammoniumhydrochlorid abfiltriert und das Filtrat im Vakuum zur Trockne gebracht.

Nach chromatographischer Reinigung und Umkristallisation aus n-Hexan werden 1,6 g (34,3 % d.Th.) farblose Kristalle vom Schmelzpunkt 88°C erhalten;

$[\alpha]_D^{21}$ : + 7,54 (c = 5, $CDCl_3$)

Analog werden enthalten

**Beispiel 2**

(R)-4-(2-n-Octyloxy-pyrimidin-5-yl)-phenyl-2,2-dimethyl-1,3-dioxolan-4-carbonsäureester

[(I) mit $R^1 = H_{17}C_8O$,

j = 1, $-A^1$ = 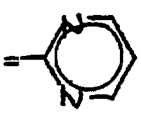,

k = l = m = null, n = 1,

$-A^3$ = 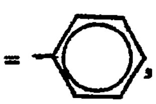,

X = O, $R^2 = R^3 = CH_3$, $R^4$ = H]

Schmelzpunkt 85°C $[\alpha]_D^{20}$ : + 7,1 (c = 1,1, $CDCl_3$)

**Beispiel 3**

(R)-4-(2-n-Octylthio-pyrimidin-5-yl)-phenyl-2,2-dimethyl-1,3-dioxolan-4-carbonsäureester

[(I) mit $R^1 = H_{17}C_8S$,

j = 1, $-A^1$ = 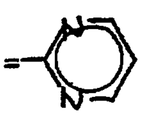,

k = l = m = null, n = 1,

$-A^3$ = 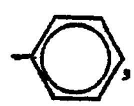,

X = O, $R^2 = R^3 = CH_3$, $R^4$ = H]
Schmelzpunkt 75°C $[\alpha]_D^{20}$ : + 7,8 (c = 1,1, $CDCl_3$)

**Beispiel 4**

(R)-4-(5-n-Octyl-pyrimidin-2-yl)-phenyl-2,2-dimethyl-1,3-dioxolan-4-carbonsäureester

[(I) mit $R^1 = H_{17}C_8$,,

j = 1, $-A^1$ = 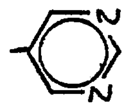,

k = l = m = null n = 1,

$-A^3$ = 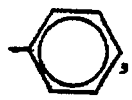,

X = O, $R^2 = R^3 = CH_3$, $R^4$ = H]
Schmelzpunkt 87°C $[\alpha]_D^{20}$ : + 9,0 (c = 5, $CDCl_3$)

**Beispiel 5**

(R)-4-(5-n-Octyloxy-pyrimidin-2-yl)-phenyl-2,2-dimethyl-1,3-dioxolan-4-carbonsäureester

[(I) mit $R^1 = H_{17}C_8O$,

j = 1, $-A^1$ = 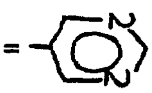,

k = l = m = null, n = 1,

$-A^3$ = 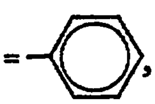,

X = O, $R^2 = R^3 = CH_3$, $R^4$ = H]
Schmelzpunkt 112°C $[\alpha]_D^{20}$ : + 11,0 (c = 5, $CDCl_3$)

**Beispiel 6**

(R)-(4'-Octyloxy-biphenyl-4-yl)-2,2-dimethyl-1,3-dioxolan-4-carbonsäureester

[(I) mit $R^1 = H_{17}C_8O$, j = k = l = m = null, n = 2,

$-A^3$ = 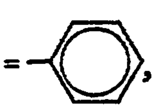,

X = O, $R^2 = R^3 = CH_3$, $R^4$ = H]
Schmelzpunkt 115°C $[\alpha]_D^{20}$ : + 7,9 (c = 1, $CDCl_3$)

**Beispiel 7**

(R),(R)-4,4'-Dihydroxydiphenyl-bis-(2,2-dimethyl-1,3-dioxolan-4-carbonsäureester)

[(I) mit $R^1$ = 2,2-Dimethyl-1,3-dioxolan-4-carbonyloxy-, j = k = l = m = null, n = 2,

$-A^3$ = 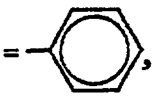,

X = O, $R^2 = R^3 = CH_3$, $R^4$ = H]
Schmelzpunkt 196°C [α]20@D: + 14,7 (c = 5, $CDCl_3$)

**Beipsiel 8**

(R)-[4-(4-Decyloxybenzoyloxy)-phenyl]-2,2-dimethyl-1,3-dioxolan-4-carbonsäureester

[(I) mit $R^1 = H_{21}C_{10}O$, j = 1,

$-A^1$ = 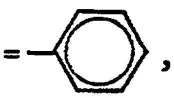,

k = l = null, m = 1,

$-M^2$ = 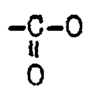 , n = 1, $-A^3$ = 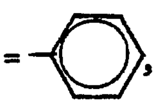,

X = O, $R^2 = R^3 = CH_3$, $R^4$ = H]
Schmelzpunkt 94°C $[\alpha]_D^{20}$ : + 5,5 (c = 2,2 $CDCl_3$)

**Beispiel 9**

(R)-4-(2-n-Octylthio-pyrimidin-5-yl)-phenyl-2,2-pentamethylen-1,3-dioxolan-4-carbonsäureester

[(I) mit $R^1 = H_{17}C_8S$, j = 1,

$-A^1$ = 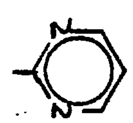,

k = l = m = null, n = 1,

$-A^3$ = 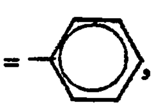,

X = O, $R^2 + R^3 = (CH_2)_5$, $R^4$ = H]
Schmelzpunkt 90,8°C $[\alpha]_D^{20}$ : + 15,1 (c = 5, $CH_2Cl_2$)

**Beispiel 10**

(R)-4-(2-n-Octyloxy-pyrimidin-5-yl)-phenyl-2,2-pentamethylen-1,3-dioxolan-4-carbonsäureester

[(I) mit $R^1 = H_{17}C_8O$, j = 1,

$-A^1 =$ 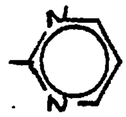,

k = l = m = null, n = 1,

$-A^3 =$ 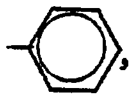,

X = O, $R^2 + R^3 = (CH_2)_5$, $R^4 = H$]
Schmelzpunkt 90,1°C $[\alpha]_D^{20}$ : + 13,6 (c = 5, $CH_2Cl_2$)

**Beispiel 11**

(R)-4-(5-n-Hexyl-pyrimidin-2-yl)-phenyl-2,2-dimethyl-1,3-dioxolan-4-carbonsäureester

[(I) mit $R^1 = H_{13}C_6$, j = 1,

$-A^1 =$ 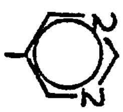,

k = l = m = null, n = 1,

$-A^3 =$ 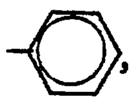,

X = O, $R^2 = R^3 = CH_3$ $R^4 = H$]
Schmelzpunkt 88,5°C $[\alpha]_D^{20}$ : + 11,0 (c = 7, $CHCl_3$)

**Beispiel 12**

(R)-4-[2-((S)-7-Methylnonyloxy)-pyrimidin-5-yl]-phenyl-2,2-dimethyl-1,3-dioxolan-4-carbonsäureester

[(I) mit $R^1 = H_5C_2CH(CH_3)(CH_2)_6O$, j = 1,

$-A^1 =$ 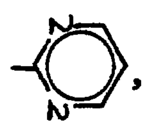,

k = l = m = null, n = 1,

$-A^3 =$ 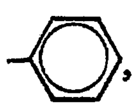,

X = O, $R^2 = R^3 = CH_3$, $R^4 = H$]
Schmelzpunkt 76°C $[\alpha]_D^{20}$ : + 10,2 (c = 5, $CH_2Cl_2$)

**Beispiel 13**

(R)-4-(5-n-Octyl-pyrimidin-2-yl)-phenyl-2,2-pentamethylen-1,3-dioxolan-4-carbonsäureester

[(I) mit $R^1 = H_{17}C_8$, j = 1,

$-A^1$ = 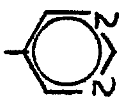,

k = l = m = null, n = 1,

$-A^3$ = 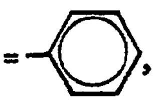,

X = O, $R^2 + R^3 = (CH_2)_5$, $R^4$ = H]
Schmelzpunkt 96,8°C $[\alpha]_D^{20}$ : + 16,6 (c = 5, $CH_2Cl_2$)

**Beispiel 14**

(R)-4-(2-n-Octyl-pyrimidin-5-yl)-phenyl-2,2-pentamethylen-1,3-dioxolan-4-carbonsäureester

[(I) mit $R^1 = H_{17}C_8$, j = 1,

$-A^1$ = 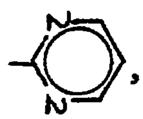,

k = l = m = null, n = 1,

$-A^3$ = 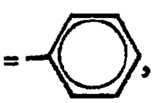,

X = O, $R^2 + R^3 = (CH_2)_5$, $R^4$ = H]
Schmelzpunkt 85,4°C $[\alpha]_D^{20}$ : + 15,0 (c = 5, $CH_2Cl_2$)

**Beispiel 15**

(R)-4-(5-n-Nonyl-pyrimidin-2-yl)-phenyl-2,2-dimethyl-1,3-dioxolan-4-carbonsäureester

[(I) mit $R^1 = H_{19}C_9$, j = 1,

$-A^1$ = 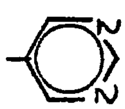,

k = l = m = null, n = 1,

$-A^3$ = 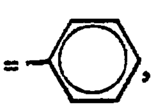,

$X = O$, $R^2 = R^3 = CH_3$, $R^4 = H$]
Schmelzpunkt 92,4°C $[\alpha]_D^{20}$: + 8,8 (c = 7,5, $CHCl_3$)

**Beispiel 16**

(R)-4-(5-n-Decyl-pyrimidin-2-yl)-phenyl-2,2-dimethyl-1,3-dioxolan-4-carbonsäureester

[(I) mit $R^1 = H_{21}C_{10}$, j = 1,

$-A^1 =$ 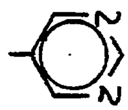 ,

k = l = m = null, n = 1,

$-A^3 =$ 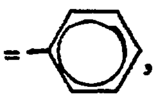 ,

$X = O$, $R^2 = R^3 = CH_3$, $R^4 = H$]
Schmelzpunkt 88°C $[\alpha]_D^{20}$: + 9,2 (c = 5, $CHCl_3$)

**Beispiel 17**

(R)-4-(5-n-Undecyl-pyrimidin-2-yl)-phenyl-2,2-dimethyl-1,3-dioxolan-4-carbonsäureester

[(I) mit $R^1 = H_{23}C_{11}$, j = 1,

$-A^1 =$ 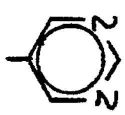 ,

k = l = m = null, n = 1,

$-A^3 =$ 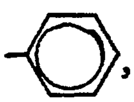 ,

$X = O$, $R^2 = R^3 = CH_3$, $R^4 = H$]
Schmelzpunkt 89,7°C $[\alpha]_D^{20}$: + 9,6 (c = 5, $CHCl_3$)

**Beispiel 18**

(R)-4-[2-(4-Hexyl-phenyl)-pyrimidin-5-yl]-phenyl-2,2-dimethyl-1,3-dioxolan-4-carbonsäureester

[(I) mit $R^1 = H_{13}C_6$, j = 1 = n = 1, k = m = null, $A^1$ =

$-A^3 =$ 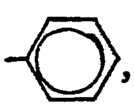 ,

$-A^2 =$ 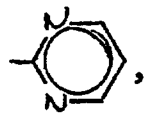,

$R^2 = R^3 = CH_3$, $R^4 = H$]
Phasenfolge K 159° $S_3$ 173° $S_C$ 190° $S_A$ 196° I
$[\alpha]_D^{20}$: + 8,7 (c = 2, $CHCl_3$)

**Beispiel 19**

(R)-4-[5-(4-Hexyl-phenyl)-pyrimidin-2-yl]-phenyl-2,2-dimethyl-1,3-dioxolan-4-carbonsäureester

[(I) mit $R^1 = H_{13}C_6$, j = l = n = l, k = m = null, $A^1$ =

$-A^3 =$ 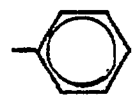, $-A^2 =$ 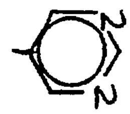,

X = O,
$R^2 = R^3 = CH_3$, $R^4 = H$]
Phasenfolge K 157° $S_3$ 162° $S_C$ 178° $S_A$ 195° I
$[\alpha]_D^{20}$: + 10,1 (c = 2, $CHCl_3$)

**Beispiel 20**

(R)-[2-(4-Dodecyloxy-phenyl)]pyrimidin-5-yl-2,2-dimethyl-1,3-dioxolan-4-carbonsäureester

[(I) mit $R^1 = H_{25}C_{12}O$, j = 1,

$-A^1 =$ 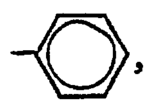,

k = l = m = null,

$-A^3 =$ 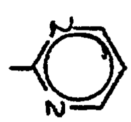,

X = O, $R^2 = R^3 = CH_3$, $R^4 = H$]
Schmelpunkt 97°C $[\alpha]_D^{20}$: + 6,9 (c = 2, $CHCl_3$)

**Beispiel 21**

(R)-[2-<4-(5-Oxo-hexyl)oxy-phenyl>]pyrmidin-5-yl-2,2-dimethyl-1,3-dioxolan-4-carbonsäureester

[(I) mit $R^1 = H_3C(C=O)(CH_2)_4O$, j = 1,

$-A^1 =$ 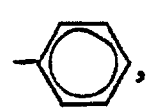,

k = l = m = null, n = 1,

$-A^3 =$ 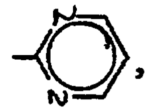,

X = O, $R^2 = R^3 = CH_3$, $R^4 = H$]
Schmelzpunkt 139°C $[\alpha]_D^{20}$: + 7,9 (c = 2, $CHCl_3$)

**Beispiel 22**

(R)-[2-<4-(4-trans-Pentylcyclohexyl)carbonyloxy-phenyl>]pyrimidin-5-yl-2,2-dimethyl-1,3-dioxolan-4-carbonsäureester

[(I) mit $R^1 = H_{11}C_5$, j = k = l = n = 1, m = null,

$-A^1 =$ 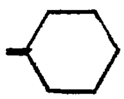, $-M^1 = -\underset{\underset{O}{\|}}{C}-O$, $-A^2 =$ 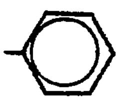, $-A^3 =$ 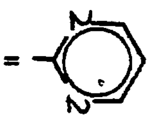,

X = O, $R^2 = R^3 = CH_3$, $R^4 = H$]
Phasenfolge K 154° N* 204° I
$[\alpha]_D^{20}$: + 6,6 (c = 2, $CHCl_3$)

**Beispiel 23**

(R)-[2-(4-Pentylcarbonyloxy-phenyl)]pyrimidin-5-yl-2,2-dimethyl-1,3-dioxolan-4-carbonsäureester

[(I) mit $R^1 = H_{11}C_5\underset{\underset{O}{\|}}{C}-O$, j = 1, $-A^1 =$ 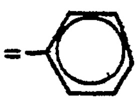,

k = l = m = null, n = 1,

$-A^3 =$ 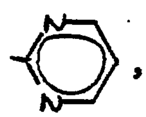,

X = O, $R^2 = R^3 = CH_3$, $R^4 = H$]
Schmelzpunkt 115,5°C $[\alpha]_D^{20}$ : +8,4 (c = 2, $CHCl_3$)

**Beispiel 24**

(R)-[4-<2-(4-Decyloxy-phenyl)methylenoxy-pyrimidin-5-yl>]phenyl-2,2-dimethyl-1,3-dioxolan-4-carbonsäureester

[(I) mit $R^1 = H_{21}C_{10}O$, j = k = l = n = 1, m = null,

$-A^1 =$ 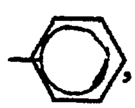, $-M^1 = -CH_2O$, $-A^2 =$ 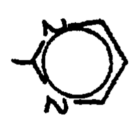, $-A^3 =$ 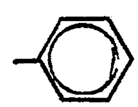,

X = O, $R^2 = R^3 = CH_3$, $R^4 = H$]
Phasenfolge K 107° $S_A$ 125° I
$[\alpha]_D^{20}$ : + 5,7 (c = 2, $CH_2Cl_2$)

**Beispiel 25**

(R)-[2-(5-Octyl-pyridin-2-yl)]phenyl-2,2-dimethyl-1,3-dioxolan-4-carbonsäureester

[(I) mit $R^1 = H_{17}C_8$, j = 1,

$-A^1$ = 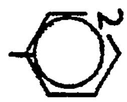,

k = l = m = null, n = 1,

$-A^3$ = 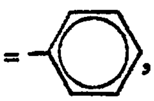,

X = O, $R^2 = R^3 = CH_3$, $R^4$ = H]
Schmelzpunkt 107°C $[\alpha]_D^{20}$ : + 10,1 (c = 2, $CHCl_3$)

**Beispiel 26**

(R)-4-[2-(4-Hexyloxy-phenyl)-pyrimidin-5-yl)phenyl-2,2-dimethyl-1,3-dioxolan-4-carbonsäureester

[(I) mit $R^1 = H_{13}C_6O$, j = l = n = 1, k = m = null,

$-A^1$ = 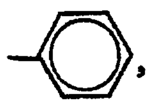, $-A^2$ = 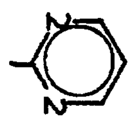, $-A^3$ = 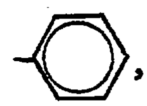, X = O,

$R^2 = R^3 = CH_3$, $R^4$ = H]
Klärpunkt 209°C $[\alpha]_D^{20}$ : + 7,9 (c = 2, $CHCl_3$)

**Beispiel 27**

(R)-[4-(4-trans-Propyl-cyclohexyl)]phenyl-2,2-dimethyl-1,3-dioxolan-4-carbonsäureester

[(I) mit $R^1 = H_7C_3$, j = 1,

$-A^1$ = 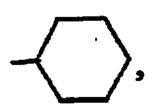,

k = l = m = null, n = 1,

$-A^3$ = 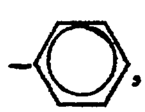,

X + O,
$R^2 = R^3 = CH_3$, $R^4$ = H]
Schmelzpunkt 99,1°C $[\alpha]_D^{20}$: +7,1 (c = 2, $CHCl_3$)

**Beispiel 28**

(R)-[4'-(4-Octyloxy-benzoyloxy)]biphenyl-4-yl-2,2-dimethyl-1,3-dioxolan-4-carbonsäureester

[(I) mit $R^1 = H_{17}C_8O$, j = k = l,

$-A^1 =$ 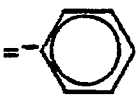,

$-M^1 = -\underset{\underset{O}{\|}}{C}-O$, l = m = null, n = 2, $-A^3 =$ 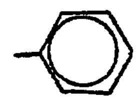,

X = O, $R^2 = R^3 = CH_3$, $R^4$ = H]
Phasenfolge K 146,5° $S_A^*$ 165,5° N* 200,5° I $[\alpha]_D^{20}$: + 5,75 (c = 2, $CHCl_3$)

**Beispiel 29**

(R)-[4-<2-(1,1-H-Perfluorooctyl)oxy-pyrimidin-5-yl>]phenyl-2,2-dimethyl-1,3-dioxolan-4-carbonsäureester

[(I) mit $R^1 = F_{15}C_7CH_2O$, j = 1,

$-A^1 =$ 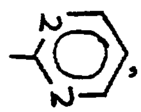,

k = l = m = null, n = 1,

$-A^3 =$ 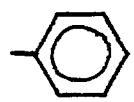,

X = O, $R^2 = R^3 = CH_3$, $R^4$ = H]
Phasenfolge K 107° $S_A$ 125° I
$[\alpha]_D^{20}$: + 3,9 (c = 2, $CH_2Cl_2$)

**Beispiel 30**

(R)-4-[5-(4-Hexyloxy-phenyl)-pyrimidin-2-yl]phenyl-2,2-dimethyl-1,3-dioxolan-4-carbonsäureester

[(I) mit $R^1 = H_{13}C_6O$, j = l = n = 1, k = m = null,

$-A^1 =$ 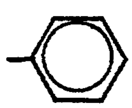, $-A^2 =$ 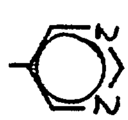, $-A^3 =$ 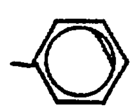, X = O,

$R^2 = R^3 = CH_3$, $R^4$ = H]
Klärpunkt 213°C $[\alpha]_D^{20}$: + 5,9 (c = 2, $CHCl_3$)

**Beispiel 31**

(R)-[4-(5-Octyl-1,3-dioxan-2-yl)-phenyl]-2,2-dimethyl-1,3-dioxolan-4-carbonsäureester

[(I) mit $R^1 = H_{17}C_8$, j = 1,

$-A^1 =$ 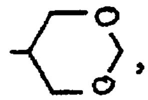,

k = l = m = null,
n = 1,

$-A^3 =$ 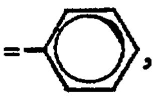,

X = O, $R^2 = R^3 = CH_3$, $R^4 = H$]
Schmelzpunkt K 104,3°C I $[\alpha]_D^{20}$: + 5,9 (c = 2, $CHCl_3$)

**Beispiel 32**

(R)-[4-(5-Octyl-1,3-dithiian-2-yl)phenyl]-2,2-dimethyl-1,3-dioxolan-4-carbonsäureester

[(I) mit $R^1 = H_{19}C_8$, j = 1,

$-A^1 =$ 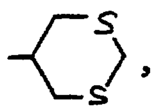,

k = l = m = null, n = 1,

$-A^3 =$ 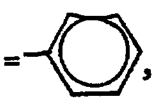,

X = O, $R^2 = R^3 = CH_3$, $R^4 = H$]
Schmelzpunkt K 129,1°C I $[\alpha]_D^{20}$: + 7,1 (c = 2, $CHCl_3$)

**Beispiel 33**

(R)-4-(5-n-Octyloxy-pyrimidin-2-yl)phenyl-2,2-pentamethylen-1,3-dioxolan-4-carbonsäureester

[(I) mit $R^1 = H_{17}C_8$, j = 1,

$-A^1 =$ 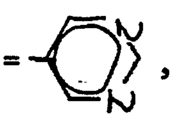,

X = 0, $R^2 + R^3 = (CH_2)_5$, $R^4$ H, k = l = m = null, n = 1,

$-A^3 =$ 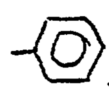]

Schmelzpunkt K 111,4° I $[\alpha]_D^{20}$: + 15,5 (c = 2, $CH_2Cl_2$)

**Beispiel 34**

(R)-[2-(4-Octyloxy-phenyl)]-pyrimidin-5-yl-2,2-pentamethylen-1,3-dioxolan-4-carbonsäureester

[(I) mit $R^1 = H_{17}C_8O$, j = 1,

$-A^1 =$ 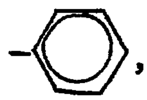,

$k = l = m = null, n = 1,$

$-A^3 =$ 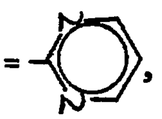,

$X = O, R^2 + R^3 = (CH_2)_5, R^4 = H]$
Schmelzpunkt K 111,6° I $[\alpha]_D^{20}$: + 16,1 (c = 2, $CHCl_3$)

**Meßmethode:**

Versetzt man ein (nicht-chirales) Lösemittel mit einer kleinen Menge einer chiralen Verbindung, so wird die Ebene des linear polarisierten Lichts um den (charakteristischen) Winkel α gedreht; dieser Winkel wird wie folgt angegeben:

$[\alpha]_D^T$ (c=x, LM), wobei die Symbole folgende Bedeutung haben: x = Konzentration der Lösung in g/l, LM = Lösemittel, D = 589 nm (NaD-Linie), T = Temperatur der Lösung. Der Drehwinkel wird in einem Polarimeter nach 10 cm Durchgang des Lichts bestimmt.

**Anwendungsbeispiele A1 bis A18**

Zur Überprüfung der Wirksamkeit der vorstehend beschriebenen Verbindungen als ferroelektrische Dotierstoffe in Flüssigkristall-Systemen mit geneigten smektischen Phasen werden diese in Konzentrationen von jeweils 10 Mol-% mit dem Racemat der Verbindung

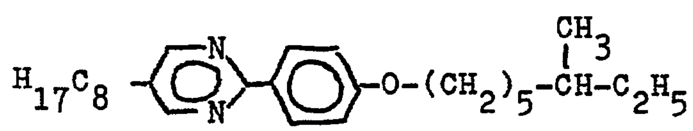 (A)

Phasenfolge:
K 14,9°C $S_C$ 49,8°C $S_A$ 59,2°C I
(5°C)
4-(5-Octyl-pyrimidin-2-yl)-1-(6-methyl-oct-1-oxy)benzol bzw. der Verbindung

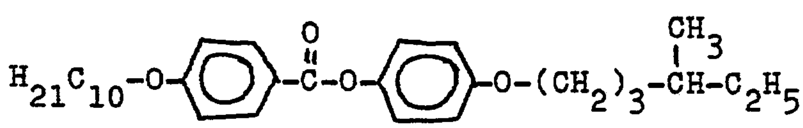 (B)

Phasenfolge:
K 17°C $S_G$ 32,7°C $S_C$ 70,4°C $S_A$ 73,3°C I
(- 3°C)
4-(4-Decyloxy-phenyl-1-carbonyloxy)-1-(4-methyl-hexyl-oxy)benzol bzw. einer nicht-chiralen Testmischung (C) mit der Phasenfolge: K 13,2°C $S_C$ 51°C $S_A$ 61,2°C N 66,7°C I bzw. einer nicht-chiralen Testmischung (D) mit der Phasenfolge K 12,5°C $S_C$ 83°C $S_A$ 95°C N 100°C I gemischt und jeweils die Werte für die spontane Polarisation ($P_S$ in $nC \cdot cm^{-2}$), für die Schaltzeit τ (in µs) und für den optischen Neigungswinkel der $S_C$-Phase θ (in °) der Mischung bestimmt. Die $P_S$-Werte werden nach der Methode von H. Diamant et al. (Rev. Sci. Instr., 28, 30, 1957) gemessen, wobei eine spezielle Meßzelle [Skarp et al. in Ferroelectric Letters Vol. 06, 67 (1986) verwendet wird, in der auch die τ- und θ-Werte bestimmt werden. Bei einer Zellenschichtdicke von ca. 2 µm wird durch Scherung eine einheitliche planare Orientierung der Flüssigkristalle in der $S_C$-Phase erreicht [SSFLC-Technik, Clark et al., Appl. Phys. Lett. 36, 899 (1980)]. Zur Bestimmung von τ und θ wird die Meßzelle auf dem Drehtisch eines Polarisationsmikroskops zwischen gekreuztem Analysator und Polarisator befestigt. Durch Drehen der Meßzelle von maximalem zu minimalem Lichtdurchgang wird der optische Neigungswinkel bzw. Schaltwinkel 2 θ, bestimmt. Mit Hilfe einer Photodiode erfolgt die Bestimmung der Schaltzeit τ, indem die Anstiegszeit des Lichtsignals von 10 auf 90 % Signalhöhe gemessen wird. Die Schaltspannung beträgt ± 10 V/µm. Neben den Werten für $P_S$,

τ, 2θ ist der $S_c$-Bereich der jeweiligen Mischung angegeben; die Werte in Klammern geben dabei die unterkühlbare untere Temperaturgrenze des $S_c$-Bereichs an. Bei Verwendung der Verbindungen (A), (B), (C) oder (D) als Wirt zur Aufnahme des Dotierstoffes beziehen sich alle Werte für $P_s$, τ und 2θ auf eine Temperatur von 25°C (40°C).

**Tabelle 1**

| (Substanz-) beispiel | Anwendungs- beispiel | Wirt | $S_c^*$-Bereich der Mischung in °C | $P_s$ nC/cm² | τ µs | 2 θ |
|---|---|---|---|---|---|---|
| 1 | A1 | A | 31[4] - 54 | 14 | 55 | 52 |
| 2 | A2 | A | 37[0] - 53 | 12,5 | 35 | 50 |
| 3 | A3 | A | 29,5[4] - 51 | 11,5 | 50 | 49 |
| 4 | A4 | A | 13[1] - 51 | 9 | 25 | 41 |
| 5 | A5 | A | 18[6] - 52 | 8 | 40 | 41 |
| 6 | A6 | A | [14] - 57 | 8,3 | 45 | 46 |
| 8 | A7 | B | 35[35] - 69 | 9,5 | 50 | 59 |
| 11 | A8 | C | 15[-8] - 43 | 9,8 | 33 | 40 |
| 13 | A9 | C | 11[-5] - 50 | 12,0 | 30 | 46 |
| 15 | A10 | C | 12[-7] - 47 | 8,6 | 25 | 40 |
| 16 | A11 | C | 15[-6] - 51 | 8,8 | 18 | 41 |
| 17 | A12 | C | 17[-6] - 50 | 7,9 | 25 | 37 |

Tabelle 1 (Forts.)

| (Substanz-) beispiel | Anwendungs- beispiel | Wirt | $S_c^*$-Bereich der Mischung in °C | $P_s$ nC/cm² | τ µs | 2 θ |
|---|---|---|---|---|---|---|
| 10 | A13 | D | 10[-6] - 78 | 26,2 | 37 | 64 |
| 12 | A14 | D | 11[-10] - 75 | 23,0 | 35 | 64 |
| 20 | A15 | D | [20] - 82 | 18,1 | 52 | 63 |
| 22 | A16 | D | | 18* | 12* | 64* |
| 25 | A17 | D | [5] - 82 | 20,2 | 76 | 61 |
| 33 | A18 | D | [10] - 80 | 22,3 | 33 | 63 |

* bei 60°C

**Anwendungsbeispiele A19 und A20**

In eine nichtchirale Flüssigkriställmischung mit der Phasenfolge I → N → $S_A$ → $S_C$ wurde jeweils eine der erfindungsgemäßen Verbindungen zugemischt und deren Verdrillungsvermögen (Erzeugung einer Helix) in der nematischen Phase untersucht. Die Bestimmung der Ganghöhe der induzierten Helix erfolgte, wie z.B. bei P. Kassubek et al., Mol. Cryst. Liq. Cryst., Vol. 8, Seite 305 bis 314, 1969 beschrieben, in einer Keilzelle mlt Orientierungsschicht durch Ausmessen der Versetzungslinien unter dem Polärisationsmikroskop. Tabelle 2 faßt die Ergebnisse zusammen.Wie aus der Tabelle zu entnehmen ist, zeigen beide Beispiele selbst bei 10 Mol-% Dotierung einen derart großen pitch in der N*-Phase, daß eine pitch-Kompensation durch einen weiteren optisch aktiven Dotierstoff nicht erforderlich ist. Beispiel A20 zeigt sogar eine Helixinversion, was bedeutet, daß die Helix ihren Drehsinn ändert und die Ganghöhe der Helix unendlich wird.

Tabelle 2

| (Substanz) beispiel | Anwendungs- beispiel | Anteil der Verbindung in Mol-% | Temperatur °C | pitch µm | Drehsinn *) der Helix | HTP **) 1/µm |
|---|---|---|---|---|---|---|
| 4 | A19 | 10 | 60 | 30 | - | - 0,33 |
| 6 | A20 | 8 | 65 | 49,3 | - | - 0,25 |
| 6 | A20 | 8 | 63,5 | ∞ | | |
| 6 | A20 | 8 | 62,4 | 46,6 | + | + 0,27 |

- (linksdrehend)
+ (rechtsdrehend)

**) HTP (helical twisting power) = $(X \cdot P)^{-1}$
X = Molenbruch des Dotierstoffs
p = pitch

**Anwendungsbeispiele A21 und A22**

Die Verbindungen der Formel (I) induzieren in orthogonalen smektischen Phasen ($S_A$, $S_B$, $S_E$) den elektroklinen Effekt, dessen Größe durch den Differentialkoeffizienten ($d\theta/dE$) angegeben wird. $\theta$ ist der vom elektrischen Feld E induzierte Neigungswinkel. Die Messung dieser Größe erfolgte in der $S^*$-Phase, jedoch in derselben "bookshelf"-Anordnung und in derselben Zelle, die auch für die Polarisationsmessungen verwendet wurde (siehe Anwendungsbeispiele A1 bis A18). Der durch das elektrische Feld induzierte Neigungswinkel wird im Polarisationsmikroskop bei gekreuzten Polarisatoren gemessen, indem man die Dunkelstellungen auffindet, die zugehörigen Winkel am Drehtisch abliest und von diesen die feldfrei gemessenen Winkel abzieht. Zur Überprüfung der Wirksamkeit der Verbindungen (I) wurde der elektrokline Koeffizient von zwei Mischungen bestimmt. Die erste (Beispiel A21) war eine Mischung des (Substanz-) Beispiels 2 (X = 0,1) in dem Racemat A (X = 0,9). Die zweite (Beispiel A22) war das (Substanz-) Beispiel 4 (X = 0,1), mit dem eine nichtchirale Grundmischung der Phasenfolge I → N → $S_A$ → $S_C$ → K dotiert wurde. Die in den so erhaltenen $S_A^*$-Phasen bestimmten Werte sind in Tabelle 3 zusammengestellt. (X = Molenbruch)

**Tabelle 3: Elektrokline Koeffizienten**

| Anwendungsbeispiel | t [°C] | ($d\theta/dE$) [$10^{-9}$ rad m/V] |
|---|---|---|
| A21 | 56 | 2,2 |
| A22 | 49 | 5,9 |
| A22 | 51 | 3,3 |
| A22 | 53 | 2,0 |

**Patentansprüche**

1. Verwendung von optisch aktiven und mesogenen 1,3-Dioxolan-4-carbonsäureestern als Dotierstoffe in Flüssigkristallmischungen, dadurch gekennzeichnet, daß 1,3-Dioxolan-4-carbonsäureester der allgemeinen Formel (I) eingesetzt werden

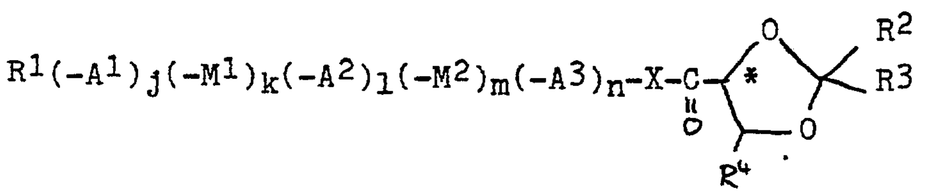 (I)

in der die Symbole folgende Bedeutung haben:

$R^1$ 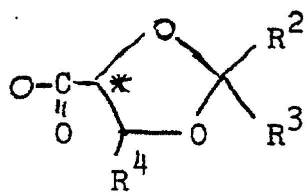

oder
ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, wobei eine oder mehrere nicht benachbarte $-CH_2$-Gruppen durch

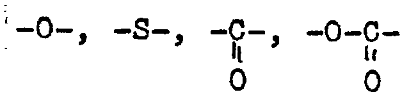

und/oder

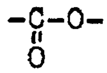

und wobei ein oder mehrere H durch F,

Cl, Br oder CN ersetzt sein können
$R^2$ und $R^3$ jeweils H oder ein Alkylrest mit 1 bis 10 C-Atomen, wobei ein oder mehrere H durch F ersetzt sein können, oder $R^2$ und $R^3$ bilden zusammen mit dem C(2)-Atom des Dioxolanrings einen Cyclopentan-, Cyclohexan- oder Cycloheptanring
$R^4$ H oder ein Alkylrest mit 1 bis 10 oder ein Alkenylrest mit 2 bis 10 C-Atomen
j und l null, 1 oder 2
k und m null oder 1
n null, 1 oder 2 mit folgender Maßgabe: wenn j und/oder l = null sind, ist k = null; wenn n = null ist, ist m = null; die Summe j + l + n ist minimal 1 und maximal 3, $-A^1$ und $-A^2$

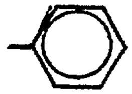 , 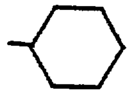 , 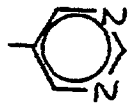 , 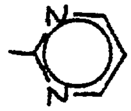 ,

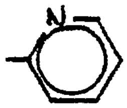 , 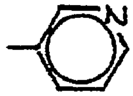 , 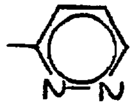 , 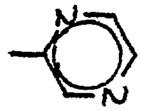 ,

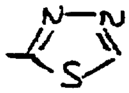 , 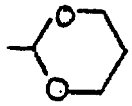 , 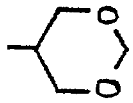 , 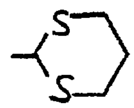 ,

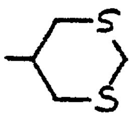 , 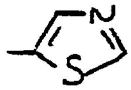 , 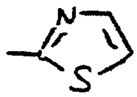 , 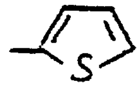 ,

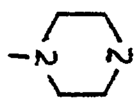

$-A^3$

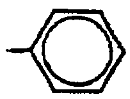 , 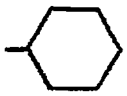 , 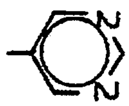 , 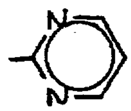 ,

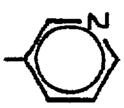 , 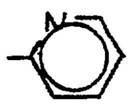 , 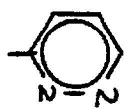 .

$-M^1$ und $-M^2$

$-\underset{\underset{O}{\Vert}}{C}-O$, $-O-\underset{\underset{O}{\Vert}}{C}$, $-CH_2CH_2$, $-CH=CH$, $-CH_2O$, $-OCH_2$

X O oder S.

2. Verwendung von optisch aktiven und mesogenen 1,3-Dioxolan-4-carbonsäureestern als Dotierstoffe in Flüssigkristallmischungen, dadurch gekennzeichnet, daß 1,3-Dioxolan-4-carbonsäureestder der allgemeinen Formel (IV) eingesetzt werden

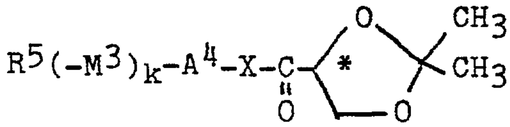 (IV)

worin bedeuten:

$R^5$ ein geradkettiger oder verzweigter Alkyl- oder Alkenylrest mit 6 bis 12 C-Atomen, der ein asymmetrisches C-Atom enthalten kann,

$-M^3$ -O, -S, 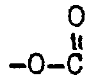 oder 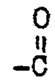

$-A^4$ 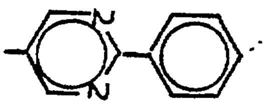 , 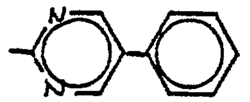 , 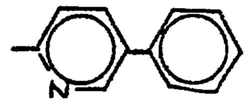 ,

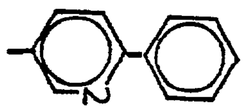 , 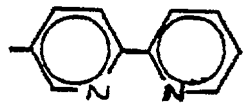 , 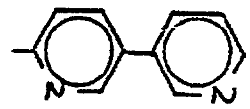 ,

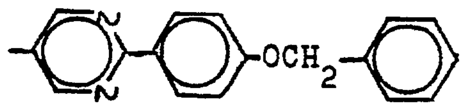 , 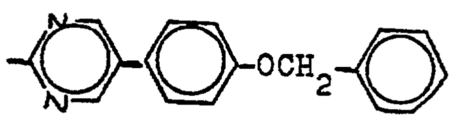

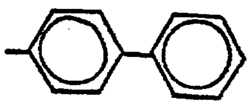 , 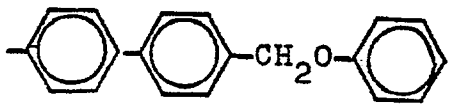 ,

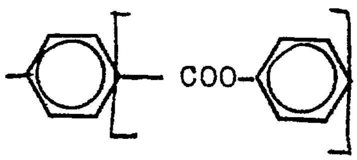 1 oder 2

3. Optisch aktive 1,3-Dioxolan-4-carbonsäureester der allgemeinen Formel (I).

4. Optisch aktive 1,3-Dioxolan-4-carbonsäureester der allgemeinen Formel (IV).

5. Flüssigkristallmischung, gekennzeichnet durch einen Gehalt an mindestens einem optisch aktiven und mesogenen 1,3-Dioxolan-4-carbonsäureester der allgemeinen Formel (I).

6. Flüssigkristallmischung, gekennzeichnet durch einen Gehalt an mindestens einem optisch aktiven und mesogenen 1,3-Dioxolan-4-carbonsäureester der allgemeinen Formel (IV).

7. Elektrooptisches Schalt- oder Anzeigeelement enthaltend eine Flüssigkristallmischung nach Anspruch 5 oder 6.

8. Verfahren zur Herstellung eines optisch aktiven 1,3-Dioxolan-4-carbonsäureesters der allgemeinen Formel (I), dadurch gekennzeichnet, daß mesogene Phenole oder Thiophenole der allgemeinen Formel (II)

$R^1(-A^1)_j(-M^1)_k(-A^2)_l(-M^2)_m(-A^3)_n$-X-H(II)

mit geeigneten Derivaten der 1,3-Dioxolan-4-carbonsäure (III), bevorzugt den Säurechloriden (mit Y = Cl)

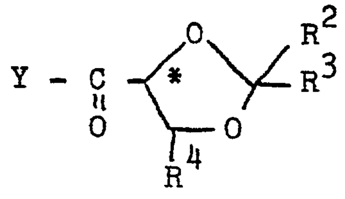 (III)

umgesetzt werden, wobei Y eine geeignete Abgangsgruppe ist.

## Claims

1. The use of an optically active, mesogenic 1,3-dioxolane-4-carboxylate as a dope in a liquidcrystal mixture, wherein a 1,3-dioxolane-4carboxylate of the general formula (I)

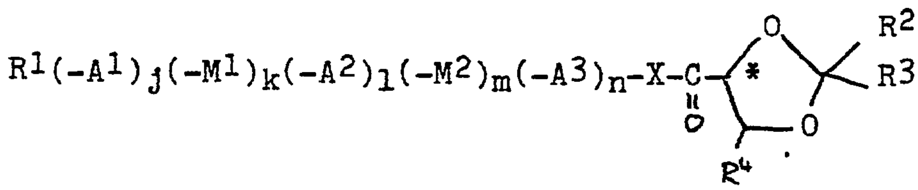 (I)

in which the symbols have the following meaning:

$R^1$ 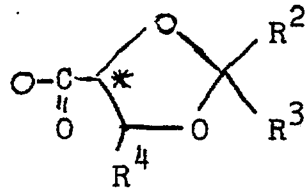

or a straight-chain or branched alkyl radical having 1 to 16 carbon atoms or a straight-chain or branched alkenyl radical having 3 to 16 carbon atoms, it being possible for these radicals themselves to contain asymmetrical carbon atoms and it being possible for one or more nonadjacent $-CH_2-$ groups to be replaced by

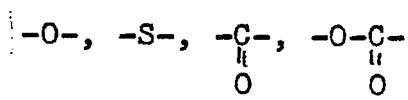

and/or

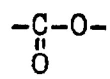

and for one or more H to be replaced by F, Cl, Br or CN,
$R^2$ and $R^3$ are each a or an alkyl radical having 1 to 10 carbon atoms, it being possible for one or more H to be replaced by F, or $R^2$ and $R^3$, together with the C(2) atom of the dioxolane ring, form a cyclopentane, cyclohexane or cycloheptane ring,
$R^4$ denotes H or an alkyl radical having 1 to 10 carbon atoms or an alkenyl radical having 2 to 10 carbon atoms,
j and l denote zero, 1 or 2,
k and m denote zero or 1,
n denotes zero, 1 or 2,
with the following proviso: if j and/or l are zero, k is zero; if n is zero, m is zero; the sum j + l + n is at least l and at most 3,
$-A^1$ and $-A^2$ denote

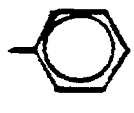 , 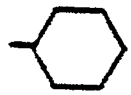 , 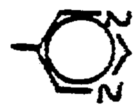 , 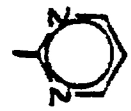 ,

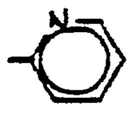 , 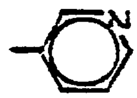 , 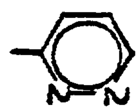 , 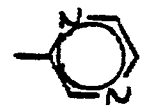 ,

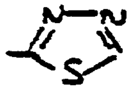 , 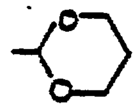 , 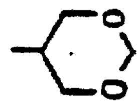 , 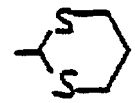 ,

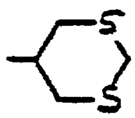 , 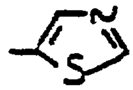 , 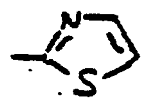 , 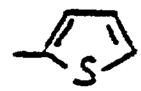 ,

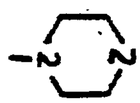

**-A[3] denotes**

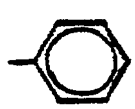 , 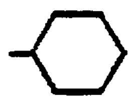 , 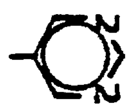 , 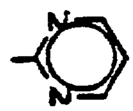 ,

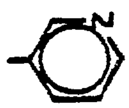 , 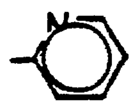 , 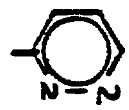

$-M^1$ and $-M^2$ denote

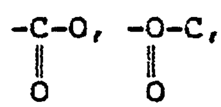

$-CH_2-CH_2-$, $-CH=CH$, $-CH_2O$, $-OCH_2$, and x denotes O or S, is employed.

2. The use of an optically active, mesogenic 1,3dioxolane-4-carboxylate as a dope in a liquid-crystal mixture, wherein a 1,3-dioxolane-4-carboxylate of the general formula (IV)

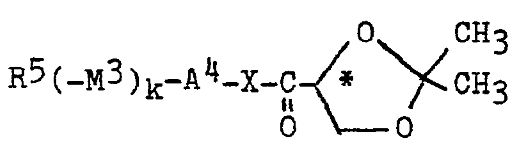 (IV)

in which:

$R^5$ denotes a straight-chain or branched alkyl or alkenyl radical which has 6 to 12 carbon atoms and which may contain an asymmetrical carbon atom,

$-M^3$ denotes -O, -S, $-O\overset{O}{\overset{\|}{C}}$ or $-\overset{O}{\overset{\|}{C}}$, and

$-A^4$ denotes

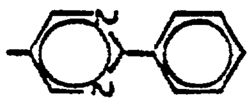 , 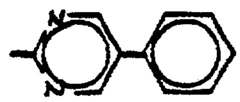 , 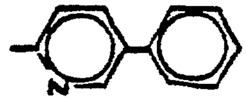 ,

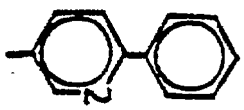 , 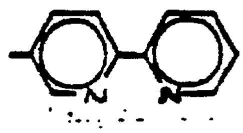 , 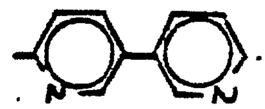 ,

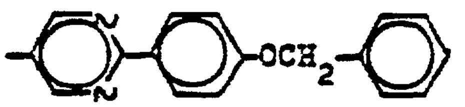 , 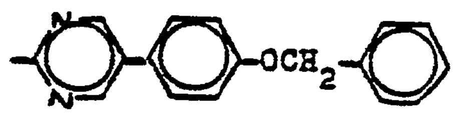 ,

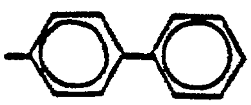 , 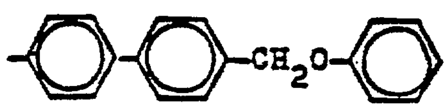 ,

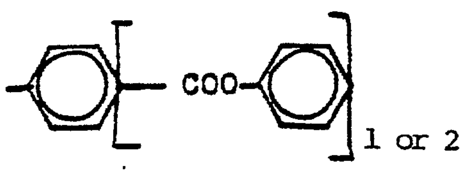

is employed.

3. An optically active 1,3-dioxolane-4-carboxylate of the general formula (I).

4. An optically active 1,3-dioxolane-4-carboxylate of the general formula (IV).

5. A liquid-crystal mixture containing at least one optically active, mesogenic 1,3-dioxolane-4-carboxylate of the general formula (I).

6. A liquid-crystal mixture containing at least one optically active, mesogenic 1,3-dioxolane-4-carboxylate of the general formula (IV).

7. An electrooptical switching or display element containing a liquid-crystal mixture as claimed in claim 5 or 6.

8. A process for the preparation of an optically active 1,3-dioxolane-4-carboxylate of the general formula (I), wherein a mesogenic phenol or thiophenol of the general formula (II)

$R^1\text{-}(\text{-}A^1)_j(\text{-}M^1)_k(\text{-}A^2)_l(\text{-}M^2)_m(\text{-}A^3)_n\text{-}X\text{-}H$ (II)

is reacted with a suitable derivative of a 1,3-dioxolane-4-carboxylic acid (III), preferably the acyl chloride (where Y = Cl)

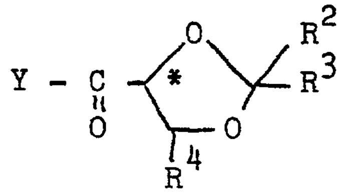 (III)

where Y is a suitable leaving group.

## Revendications

1. Utilisation d'esters optiquement actifs et mésogènes d'acide dioxolanne-1,3 carboxylique-4 en tant que dopants dans des mélanges mésomorphes, caractérisée en ce qu'on utilise des esters d'acide dioxolanne-1,3 carboxylique-4 de formule générale (I)

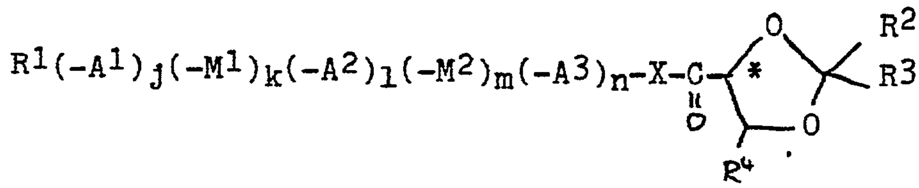
(I)

où les symboles ont les significations suivantes:

$R^1$ est

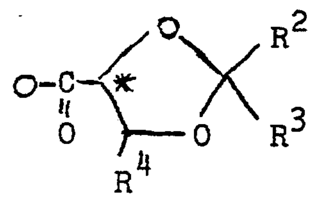

ou un radical alkyle à chaîne droite ou ramifiée ayant de 1 à 16 atomes de carbone ou un radical alcényle à chaîne droite ou ramifiée ayant de 3 à 16 atomes de carbone, ces radicaux pouvant eux-mêmes contenir des atomes de carbone asymétriques, un ou plusieurs groupes $-CH_2-$ non voisins pouvant être remplacés par

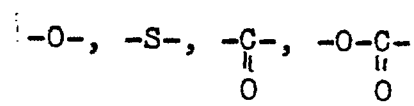

un ou plusieurs hydrogènes pouvant être remplacés par F, Cl, Br ou CN,

$R^2$ et $R^3$ représentent chacun un hydrogène ou un radical alkyle ayant de 1 à 10 atomes de carbone, un ou plusieurs hydrogènes pouvant être remplacés par H, ou encore $R^2$ et $R^3$ forment, avec l'atome C(2) du noyau dioxolanne, un noyau cyclopentane, cyclohexane ou cycloheptane,

$R^4$ est H ou un radical alkyle ayant de 1 à 10 atomes de carbone ou un radical alcényle ayant de 2 à 10 atomes de carbone,

j et l valent chacun 0, 1 ou 2,

k et m valent chacun 0 ou 1,

n vaut 0, ou 2,

à la condition que, quand j et/ou l sont chacun nuls, k vaille zéro; quand n vaut zéro, m vaille zéro; et que la somme j + l + n vaille au minimum 1 et au maximum 3,

$-A^1$ et $-A^2$ représentent:

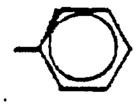, 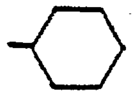, 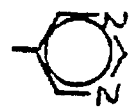, 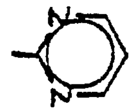,

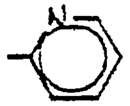, 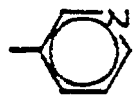, 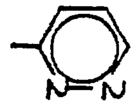, 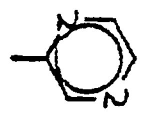,

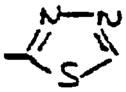, 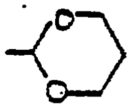, 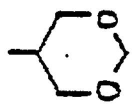, 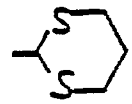,

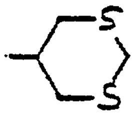, 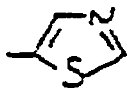, 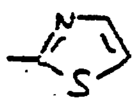, 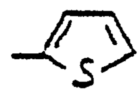,

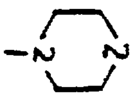

$-A^3$ représente

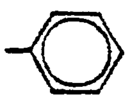, 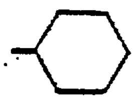, 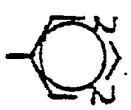, 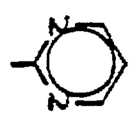,

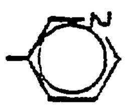, 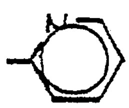, 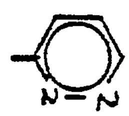

$-M^1$ et $-M^2$ représent:

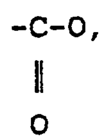, 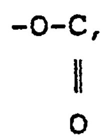,

$-CH_2-CH_2-$, $-CH=CH$, $-CH_2O$, $-OCH_2$,
X représente O ou S.

2. Utilisation d'esters optiquement actifs et mésogènes d'acide dioxolanne-1,3 carboxylique-4 en tant que dopants dans des mélanges mésomorphes, caractérisée en ce qu'on utilise des esters d'acide dioxolanne-1,3 carboxylique-4 de formule générale (IV)

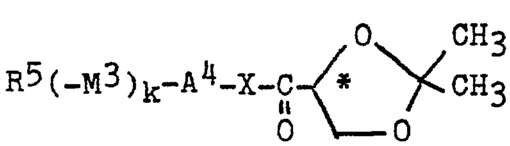 (IV)

dans laquelle

$R^5$ est un radical alkyle ou alcényle à chaîne droite ou ramifiée ayant de 6 à 12 atomes de carbone, qui peut contenir un atome de carbone asymétrique,

$-M^3$ est -O, -S, $-O-\overset{\overset{O}{\|}}{C}$ ou $-\overset{\overset{O}{\|}}{C}$

$-A^4$

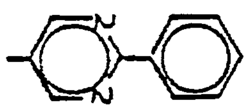 , 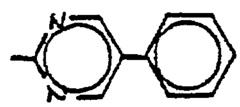 , 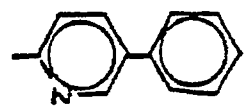 ,

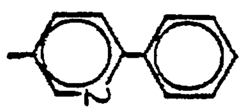 , 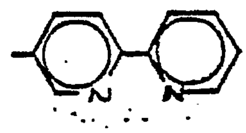 , 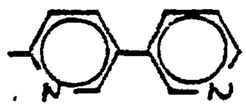 ,

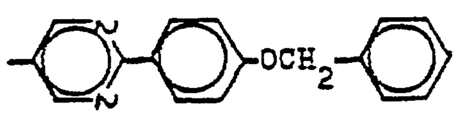 , 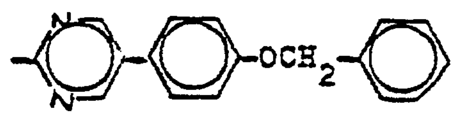 ,

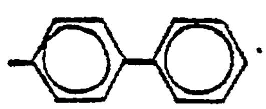 , 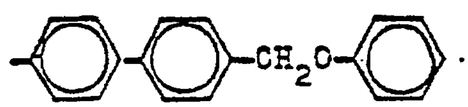 ,

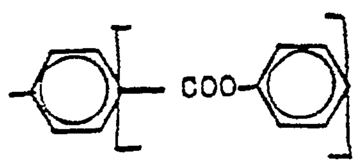 1 ou 2

3. Esters optiquement actifs d'acide dioxolanne-1,3 carboxylique-4 de formule générale (I).

4. Esters optiquement actifs d'acide dioxolanne-1,3 carboxylique-4 de formule générale (IV).

5. Mélange mésomorphe, caractérisé en ce qu'il contient au moins un ester optiquement actif et mésogène d'acide dioxolanne-1,3 carboxylique-4 de formule générale (I).

6. Mélange mésomorphe, caractérisé en ce qu'il contient au moins un ester optiquement actif et mésogène d'acide dioxolanne-1,3 carboxylique-4 de formule générale (IV).

7. Elément de commutation et d'affichage électrooptique contenant un mélange mésomorphe selon les revendications 5 ou 6.

8. Procédé pour la préparation d'un ester optiquement actif d'acide dioxolanne-1,3 carboxylique-4 de formule générale (I), caractérisé en ce qu'on fait réagir des phénols ou thiophénols mésogènes de formule générale (II)

$R^1(-A^1)_j(-M^1)_k(-A^2)_l(-M^2)_m(-A^3)_n-X-H$ (II)

avec des dérivés appropriés de l'acide dioxolanne-1,3 carboxylique-4 (III), de préférence les chlorures d'acide (avec Y = Cl)